# EUROPEAN PATENT SPECIFICATION

(11) **EP 2 433 952 B1**
(45) Date of publication and mention of the grant of the patent: **02.09.2015**
(21) Application number: 11250813.0
(22) Date of filing: 20.09.2011
(51) Int. Cl.: C07K 1/18, B01D 15/16, B01D 15/36

(54) **Liquid chromatography system and method for protein separation and purification**
Flüssigkeitschromatographiesystem und Verfahren zur Proteintrennung und -reinigung
Système de chromatographie liquide et procédé pour la séparation et la purification de protéines

(30) Priority: 20.09.2010 US 403741 P
(43) Date of publication of application: 28.03.2012
(73) Proprietor: Asahi Kasei Bioprocess, Inc., Glenview, IL 60026 (US)
(72) Inventor: Li, Michael, Glenview Illinois 80026 (US); Yabe, Hiroyuki, Glenview Illinois 80026 (US); Miyabayashi, Tomoyuki, Glemview Illinois 60026 (US)
(74) Representative: Dauncey, Mark Peter

(56) References cited:
- WO-A2-2004/103519
- US-A1- 2008 279 038
- AHAMED ET AL: "pH-gradient ion-exchange chromatography: An analytical tool for design and optimization of protein separations", JOURNAL OF CHROMATOGRAPHY, ELSEVIER SCIENCE PUBLISHERS B.V, NL, vol. 1164, no. 1-2, 23 August 2007 (2007-08-23), pages 181-188, XP022209212, ISSN: 0021-9673, DOI: 10.1016/J.CHROMA.2007.07.010

## Description

### CLAIM OF PRIORITY

This application claims priority to provisional patent application no. 61/403,741, filed September 20,2010, currently pending.

### FIELD OF THE INVENTION

The present invention generally relates to liquid chromatography systems and methods and, more particularly, to a system and method where acid and base/alkali liquids are blended in an inline buffer dilution device and in accordance with a method to provide a linear pH gradient during delivery of the blended liquid to a chromatography column loaded with a protein solution for protein separation and purification.

### BACKGROUND

Biopharmaceutical manufacturing requires buffer solutions for critical downstream processes. Chromatography is an integral part of the downstream bioprocessing steps, and the buffered mobile phase properties, such as ionic strength and pH. are often critical process parameters for protein purification. Traditionally, a salt gradient is used to separate proteins with different ionic characteristics. Using this approach, however, protein recovery may be poor and the de-salting steps cumbersome and time-consuming.

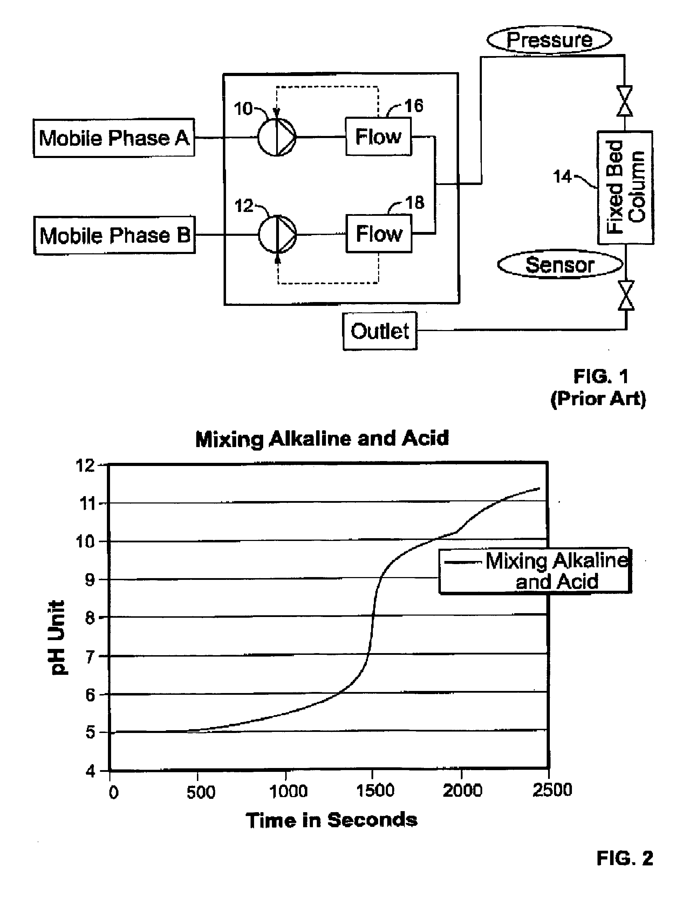

Protein separation by pH gradient using prior art systems has proven to be difficult and unpopular. For example, with reference to Fig. 1, a prior art liquid chromatography system consists of two pumps 10 and 12 that independently deliver mobile phase A (acid) and B (base or alkali) to a liquid chromatography column 14. As a result, the percentages of mobile A and mobile B in the blended liquid entering the chromatography column is dependent on the accuracy of the system pumps. The pumps are controlled solely by feedback of the flow rate of the liquids exiting the pumps as detected by flow meters 16 and 18 of Fig. 1.

Use of the prior liquid chromatography system of Fig. 1 generates the sigmoid titration curve of Fig. 2. As illustrated in Fig. 2, during the change of the blended liquid from the acidic to alkaline condition, and vice versa, a steep slope is formed (around 1500 seconds in Fig. 2). Because of the sharp rise in pH during the process, it is very difficult to control the elution characteristics of proteins loaded in the column 14. More specifically, because the titration curve between acid and alkali, whether strong or weak, is not linear, protein chemists are reluctant to use a pH gradient during chromatographic runs to resolve proteins with similar isoelectric points (pI).

In view of the above, and as noted previously, proteins are conventionally purified and separated in a liquid chromatography column by salt gradient (conductivity) and not by pH gradient. Use of conductivity, however, often results in great variances from what is desired. This is often due to human variances in making the buffer solution or variances due to feed stocks from suppliers (for example, 5% off in the salt concentration). Use of a pH gradient in purification/separation of proteins would provide greater control and accuracy, if the above sigmoid titration curve issues could be overcome. It is therefore desirable to make linear pH gradients an option for purifying proteins available, for example, in the biopharmaceutical industry.

### BRIEF DESCRIPTION OF THE DRAWINGS

Fig.1 is a schematic view of a prior art liquid blending and chromatography system;
Fig. 2 is a graph illustrating sigmoid titration curve formed by mixing an acid and an alkali using the prior art system of Fig. 1;
Fig. 3 is schematic view of an embodiment of the liquid chromatography protein separation system of the present invention;
Fig. 4 is a graph illustrating a comparison of titration curves produced by the systems of Figs. 1 and 3;
Fig. 5 is a graph illustrating elution behavior of two proteins with similar pI under different pH gradient influences from the systems of Figs.1 and 3; and
Fig. 6 is a graph illustrating a comparison of curves using a two-solvent embodiment of the system and method of the present invention.

### DETAILED DESCRIPTION OF EMBODIMENTS

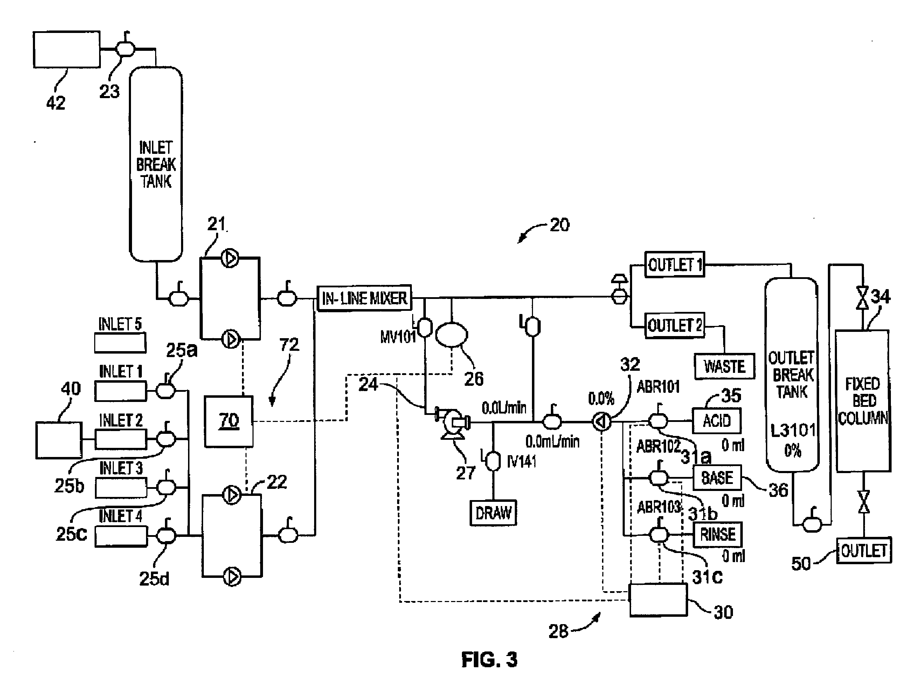

In accordance with the present invention, an accurate blending module or inline buffer dilution device (IBD), such as the one indicated in general 20 in Fig. 3, and in commonly assigned U.S. Patent No. 7,515,994 to Bellafiore et al. can be used to produce a linear pH gradient in blended liquids for protein separation and purification from a protein solution loaded in a liquid chromatography column. U.S. Patent No. 7,515,994, and related U.S. Patent No. 7,072,742 and U.S. Patent Application Publication No. US 2008/0279038, are cited herein. Document WO 2004103519 discloses a system in which a controller unit monitors the pH of a solution in a flow cell by adjusting the actual pH to the desired pH via acceleration/deceleration of pumps according to the flow control method.

Fig. 3 shows a piping and instrumentation diagram of the IBD device 20 in an embodiment of the system of the invention. Either an acid or base/alkali is connected to primary pump 21, and the other to concentrate pump 22. A primary inlet valve 23 communicates with the inlet of the primary pump 21, which may be a water for injection (WFI) pump. A number of concentrate inlet valves 25a-25d communicate with the inlet of the concentrate pump 22. The acidic and alkaline solutions are pumped into a mixing loop 24, which preferably features recirculation pump 27, where the resulting titration solution can be instantaneously analyzed by a controller 30 via a pH sensor 26, so that an inline process pH analyzer is formed. The sensor 26 interfaces with the controller 30 which interfaces with automated titration valves 31a-31c and a titration pump 32 so that a process control feedback loop, indicated in general at 28 in Fig. 3, is formed.

Controller 30 may be, as an example only, a programmable logic controller (PLC). In accordance with the user-defined pH parameters, the controller 30, via the pH sensor 26, continually monitors and adjusts the acidity of the solution in mixing loop 24 by manipulating the automated titration valves 31a-31c and controlling the titration pump 32 so as to pump respective acid or base to the mixing loop. Consequently, a generally linear pH gradient can be created to satisfy the user's requirements by operation of the titration pump 32 and configuration of valves 31a-31c. In accordance with an embodiment of the method of the present invention, the user requirements are to provide a linear pH gradient during delivery of the blended liquid to liquid chromatography column 34 for protein separation and purification from a protein solution loaded in the column.

Controller 30 is preferably configured as a proportional-integral-derivative (PID) controller to control the titration valves 31a-31c and the titration pump 32 and to incorporate the user-defined set points into the IBD device 20 via the controller software. As is known in the art, by timing the three parameters in the PID controller algorithm, the controller can provide control action designed for specific process requirments. The response of the controller can be described in terms of the responsiveness of the controller to an error, the degree to which the controller overshoots the set point and the degree of system oscillation.

In accordance with an embodiment of the system and method of the present invention, the IBD device 20 of Fig. 3 is connected to a chromatography column 34 packed with a cation exchange resin (available, for example, from Tosoh biosciences). As an example only, the chromatography column may have a 10cm inner diameter. It should be understood that an anion exchange resin may be used as an alternative to the cation resin (i.e. any ion exchange resin may be used in the invention). As explained in greater detail below, in an embodiment of the invention, acetic acid (available, for example, from Amresco) and ammonium hydroxide (available, for example, from JT Baker) are used as the acid and base/alkali, 40 and 42 in Fig. 3, respectively, to separate and purify two proteins, for example, Ribonuclease A and Trypsinogen (available, for example, from Worthington Enzymes and Sigma Aldrich, respectively).

Using the IBD device 20 of Fig. 3, a supply of an acetic acid (20 mM) solution 40 is connected to the concentrate pump 22 via inlet valve 25b, which is opened, and the concentrate pump 22 is run with the same starting and ending pump frequency of 12.3%, A supply of ammonium hydroxide (10 mM) 42 is connected to the primary pump 21, via inlet valve 23, which is opened, and the primary pump 21 is operated with a starting pump frequency of 14% and an ending frequency of 10%. Aciditly of the solution is continually adjusted, via pH sensor 26 and controller 30, with ammonium hydroxide (100 mM), a supply of which is indicated at 36 in Fig. 3, delivered from the titration pump 32 with a starting pump frequency of 10% and ending with 100%. Titration valve 31 b is open, but valves 31 a and 31 c are closed. In the first five minutes, the pH of the blend is maintained at 6 followed by a linear gradient run to pH 10.3 over 30 minutes.

It should be noted that the starting and ending pump frequencies are dependent on pump strength, which are 300 liters/hour for primary pump 21 and 150 liters/hour for concentrate pump 22 in the embodiment illustrated. For example, if the strength of primary pump 21 were changed from 300 liters/hour to 400 liters/hour, a new pump frequency would be required to obtain a linear pH gradient. In addition, if the molarity of the solution were changed, for example, from 20 mM to 40 mM, the pump frequency would also have to be adjusted.

To provide a comparison to the method of the above paragraph, a pH gradent was generated using the IBD device of Fig. 3, but without using the feedback loop 28 and titration pump 32. More specifically, the orientation of the pumps 21 and 22 and the concentration of the mobile phases was the same as the linear pH gradient of the above paragraph, except that the feedback control for pH adjustment was idle. While the starting frequency of the concentrate pump 22 was 29.3% and 7.39% at the end, the primary pump 21 was raised from the initial 10% to final 29.45% over 40 minutes. The initial pH was just below 5, and the final pH was about 11.

Of the two proteins, Trypsinogen, has an isoelectric point (pI) equal to 8.3, and Ribonuclease A, has a pI equal to 9.4. For both of the above runs, 50 mg of each protein were dissolved in 20 mM acetic acid, pH 4, and 100 mM sodium chloride. The mixture was loaded onto the column 34 packed with cation exchange resins. Before loading, the column was pre-equilibrated with a pH 6 starting buffer.

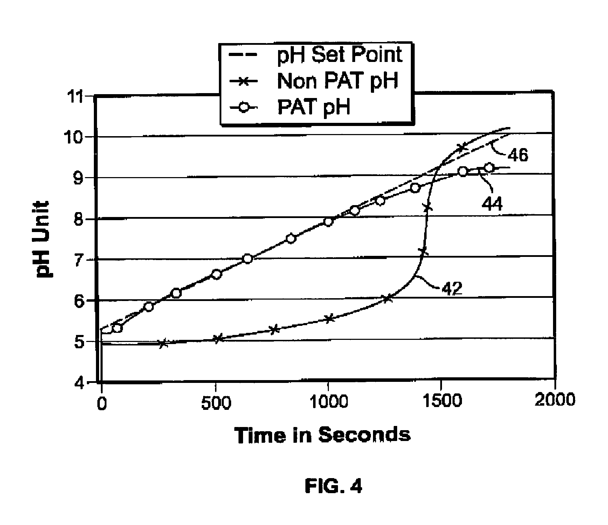

As stated previously, in each run, the 20 mM acetic acid, a weak acid, was titrated with 10 mM ammonium hydroxide, a weak base, to create a pH curve. Fig. 4 shows the results of the two runs. Simply blending acid and alkali without pH adjustment and control via the feedback loop 28 and titration pump 32 (i.e, a passive IBD device) a typical sigmoid titration curve was generated as shown by the X-line 42 in Fig. 4. The steep rise of the pH value from 6.5 to 9.5 makes it difficult to resolve proteins whose pl would be within this range. In contrast, active use of the IBD device 20, that is, using the feedback loop 28 and titration pump 32. was able to develop, and deliver to column 34, a linear pH gradient, illustrated by O-line 44 in Fig. 4. The interfaced process control feedback loop 28 in the active IBD device 20 method constantly analyzed and adjusted the pH of the solution in the loop 24, and referenced the current process value to the user-defined pH target set point in the controller 30 at any given time (line 46 in Fig. 4). More specifically, the controller 30, based on readings from the pH sensor 26, sent a signal to adjust the speed of the titration pump 32 so that the dynamic pH value in the mixing loop 24 was continually adjusted towards the pre-determined set-point.

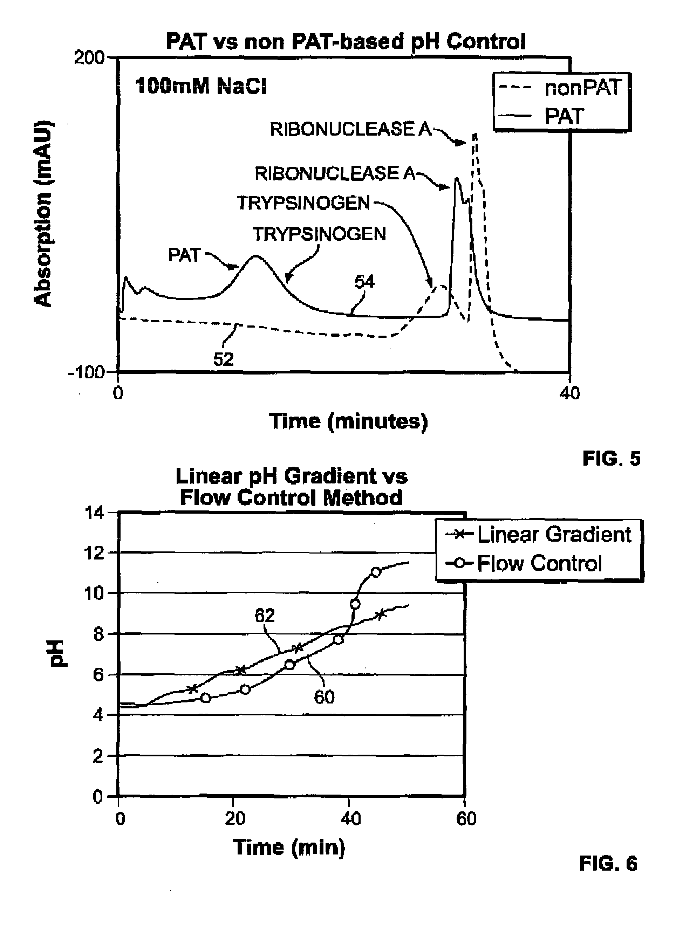

As noted above, two proteins, Trypsinogen and Ribonuclease A, with similar pI, were chosen to illustrate the effectiveness of the pH control in the active IBD device system and method, which is best shown in Fig. 5, where the elusion behaviors of the two proteins are illustrated. In the case of the passive IBD device 20 operation, the sharp rise of pH from 6.5 to 95 within a few minutes caused both proteins to be de-sorbed and to elute from the column almost together through outlet 50 (Fig. 3). As a result, resolution was not good as evidenced by the line 52 in Fig. 5. On the other hand, the gentle pH increase through the active IBD device pH control selectively and slowly changed the ionic characters of the two proteins. As a result, both proteins were baseline separated through outlet 50 as shown by line 54 in Fig. 4.

The embodiment of the system and method of the present invention illustrated in the figures and described above, that is where an active IBD device was used, was able to produce a generally linear pH gradient. By having an inline process pH analyzer (pH sensor 26 and controller 30) included within process control feedback loop 28, two proteins, Trypsinogen and Ribonuclease A, were successfully resolved. The technology of the present invention could be extended to separate other proteins having pl similar to one another or to remove high molecular weight aggregates from the parent monomer.

An example of the results of such a two-liquid approach is provided in Fig. 6. In the two-solvent system used to generate Fig. 6, acetic acid (40mM), pH 4, is provided via primary pump 21, and trisodium phosphate (20mM), pH 12, is provided via concentrate pump 22. To achieve the "Linear Gradient", illustrated in the graph of Fig. 6 at 62, the solvents from pumps 21 and 22 are pumped into the mixing loop 24, where the resulting solution is instantaneously analyzed by a controller 70 connected to pH sensor 26 to form an inline process pH analyzer. As illustrated in Fig. 3, controller 70, which also is preferably configured as a PID controller, interfaces with and controls pumps 21 and 22 so that a process control feedback loop, indicated in general at 72 in Fig. 3, is formed. In accordance with the user-defined pH parameters, the controller 70, via the pH sensor 26, continually monitors and adjusts the acidity of the solution by controlling pumps 21 and 22 to provide acid or base. Consequently, a generally linear pH gradient is created to satisfy the user's requirements by operation of the pumps 21 and 22.

To provide a comparison to the method of the above paragraph, in a "Flow Control" mode of operation, a pH gradent was generated using the IBD device 20 of Fig. 3, but without using the pH sensor 26 or controller 70. More specifically, the orientation of the pumps 21 and 22 and the concentration of the mobile phases was the same as the linear pH gradient run of the above paragraph, except that the feedback control for pH adjustment was idle. As a result, the "Flow Control" curve of Fig. 6, illustrated at 60, was generated. The steep slope starting from pH 8 and rising to pH I1 over 2 minutes for curve 60 should be noted in Fig. 6.

In generating the Linear Gradient result (62) of Fig. 6. primary pump 21 was operated with a starting frequency of 24% and an ending frequency of 10%, while concentrate pump 22 was operated with a start frequency of 10% and an ending frequency of 67% (with a combined flow rate of the two pumps equal to 0.4 L/min.). To generate the Flow Control result (60) of Fig. 6, primary pump 21 was operated with a starting frequency of 64% and an ending frequency of 10%, while concentrate pump 22 was operated with a start frequency of 5% and an ending frequency of 67% (with a combined flow rate of the two pumps equal to 0.4 L/min.).

## Claims

1. A system for protein separation and purification comprising:
a. a mixing device;
b. a primary pump having an inlet in communication with a supply of an acid, an alkali or water and an outlet in communication with the mixing device;
c. a concentrate pump having an inlet in communication with a supply of an acid or an alkali and an outlet in communication with the mixing device;
d. a liquid chromatography column in communication with the mixing device and packed with an ion exchange resin and loaded with proteins;
e. a process control feedback loop in communication with the mixing device, the process control feedback loop including a controller that interfaces with a titration pump and an inline process pH analyzer, the titration pump having an inlet in communication with a supply of acid or alkali and an outlet in communication with the mixing device;
whereby the controller monitors the pH of a solution in the mixing device via the pH analyzer and adjusts the acidity of the solution by controlling the titration pump to pump acid or alkali from the supply of acid or alkali into the mixing device to provide a generally linear pH gradient to the chromatography column so that proteins are separately eluted from the liquid chromatography column.

2. The system of claim 1 further comprising a recirculation pump in the mixing device.

3. The system of claim 1 wherein the process control feedback loop includes a plurality of automated titration valves in communication with the inlet of the titration pump that are controlled by the controller.

4. The system of claim 1 wherein the primary pump is a WFI pump.

5. The system of claim 3 wherein the titration valves are in communication with at least one of the primary pump or the concentrate pump so that the pH of the titration solution in the mixing device is adjusted by acid or alkali from the primary pump and/or acid or alkali from the concentrate pump.

6. The system of claim 1 wherein the controller is a programmable logic controller or is configured as a PID controller.

7. A method for separating and purifying proteins comprising the steps of:
a. providing a mixing device, a primary pump in communication with the mixing device, a concentrate pump in communication with the mixing device, a liquid chromatography column in communication with the mixing device and a process control feedback loop in communication with the mixing device including a controller that interfaces with a titration pump and an inline process pH analyzer, the titration pump having an inlet in communication with a supply of acid or alkali and an outlet in communication with the mixing device:
b. packing the liquid chromatography column with an ion exchange resin;
c. loading the chromatography column with a protein solution containing proteins;
d. supplying an acid or an alkali to the mixing device using the primary pump;
e. supplying an acid or an alkali to the mixing device using the concentrate pump so that the solution is created in the mixing device; and
f. adjusting the pH of the solution in the mixing device using the controller of the feedback loop, whereby the controller monitors the pH of a solution in the mixing device via the pH analyzer and adjusts the acidity of the solution by controlling the titration pump to pump acid or alkali from the supply of acid or alkali into the mixing device to provide a generally linear pH gradient to the chromatography column so that proteins are separately eluted from the liquid chromotography column.

8. The method of claim 7 wherein the process control feedback loop includes a plurality of automated titration valves in communication with the inlet of the titration pump that are controlled by the controller.

9. The method of claim 8 wherein the titration valves are in communication with at least one of the primary pump of the concentrate pump and wherein the titration solution in step f. is adjusted by acid or alkali provided by the primary pump and/or the concentrate pump.

10. The method of claim 7 wherein a frequency of one of the primary pump or the concentrate pump is held steady during steps d. and e. while a frequency of the other one of the primary pump or the concentrate pump is varied during steps d. and e.

11. The system or method of any preceding claim wherein the mixing device includes a mixing loop.

12. The system or method of any preceding claim wherein the proteins includes at least two proteins having a first isoelectric point and a second protein having a second isoelectric point.

## Patentansprüche

1. System zur Proteintrennung und -reinigung, umfassend:
a. eine Mischvorrichtung;
b. eine Primärpumpe, die einen Einlass in Kommunikation mit einem Vorrat von einer Säure, einem Alkali oder Wasser und einen Auslass in Kommunikation mit der Mischvorrichtung aufweist;
c. eine Konzentratpumpe, die einen Einlass in Kommunikation mit einem Vorrat von einer Säure oder einem Alkali und einen Auslass in Kommunikation mit der Mischvorrichtung aufweist;
d. eine Flüssigchromatographiesäule in Kommunikation mit der Mischvorrichtung und gepackt mit einem Ionenaustauschharz und beladen mit Proteinen;
e. eine Prozessregelungs-Rückkopplungsschleife in Kommunikation mit der Mischvorrichtung, wobei die Prozessregelungs-Rückkopplungsschleife einen Controller einschließt, der eine Schnittstelle mit einer Titrationspumpe und einer Inline-Prozess-pH-Analysevorrichtung hat, wobei die Titrationspumpe einen Einlass in Kommunikation mit einem Säure- oder Alkalivorrat und einen Auslass in Kommunikation mit der Mischvorrichtung aufweist;
wodurch der Controller den pH-Wert einer Lösung in der Mischvorrichtung via die pH-Analysevorrichtung überwacht und die Acidität der Lösung einstellt durch Regeln der Titrationspumpe so, dass sie Säure oder Alkali aus dem Säure- oder Alkalivorrat in die Mischvorrichtung pumpt, um einen im Allgemeinen linearen pH-Gradienten für die Chromatographiesäule bereitzustellen, so dass Proteine separat aus der Flüssigchromatographiesäule eluiert werden.

2. System nach Anspruch 1, das weiterhin eine Rezirkulationspumpe in der Mischvorrichtung umfasst.

3. System nach Anspruch 1, wobei die Prozessregelungs-Rückkopplungsschleife mehrere automatisierte Titrationsventile in Kommunikation mit dem Einlass der Titrationspumpe einschließt, die durch den Controller geregelt werden.

4. System nach Anspruch 1, wobei die Primärpumpe eine WFI-Pumpe ist.

5. System nach Anspruch 3, wobei die Titrationsventile in Kommunikation mit zumindest einer von der Primärpumpe oder der Konzentratpumpe sind, so dass der pH-Wert der Titrationslösung in der Mischvorrichtung durch Säure oder Alkali aus der Primärpumpe und/oder durch Säure oder Alkali aus der Konzentratpumpe eingestellt wird.

6. System nach Anspruch 1, wobei der Controller ein programmierbarer logischer Controller ist oder als PID-Controller konfiguriert ist.

7. Verfahren zur Trennung und Reinigung von Proteinen, umfassend die Schritte:
a. Bereitstellen einer Mischvorrichtung, einer Primärpumpe in Kommunikation mit der Mischvorrichtung, einer Konzentratpumpe in Kommunikation mit der Mischvorrichtung, einer Flüssigchromatographiesäule in Kommunikation mit der Mischvorrichtung und einer Prozessregelungs-Rückkopplungsschleife in Kommunikation mit der Mischvorrichtung, einschließlich eines Controllers, der eine Schnittstelle mit einer Titrationspumpe und einer Inline-Prozess-pH-Analysevorrichtung hat, wobei die Titrationspumpe einen Einlass in Kommunikation mit einem Säure- oder Alkalivorrat und einen Auslass in Kommunikation mit der Mischvorrichtung aufweist;
b. Packen der Flüssigchromatographiesäule mit einem Ionenaustauschharz;
c. Laden der Chromatographiesäule mit einer Proteine enthaltenden Proteinlösung;
d. Zuführen einer Säure oder eines Alkalis zur Mischvorrichtung unter Verwendung der Primärpumpe;
e. Zuführen einer Säure oder eines Alkalis zur Mischvorrichtung unter Verwendung der Konzentratpumpe, so dass die Lösung in der Mischvorrichtung erzeugt wird; und
f. Einstellen des pH-Werts der Lösung in der Mischvorrichtung unter Verwendung des Controllers der Rückkopplungsschleife, wodurch der Controller den pH-Wert einer Lösung in der Mischvorrichtung via die pH-Analysevorrichtung überwacht und die Acidität der Lösung einstellt durch Regeln der Titrationspumpe so, dass sie Säure oder Alkali aus dem Säure- oder Alkalivorrat in die Mischvorrichtung pumpt, um einen im Allgemeinen linearen pH-Gradienten für die Chromatographiesäule bereitzustellen, so dass Proteine separat aus der Flüssigchromatographiesäule eluiert werden.

8. Verfahren nach Anspruch 7, wobei die Prozessregelungs-Rückkopplungsschleife mehrere automatisierte Titrationsventile in Kommunikation mit dem Einlass der Titrationspumpe einschließt, die durch den Controller geregelt werden.

9. Verfahren nach Anspruch 8, wobei die Titrationsventile in Kommunikation mit zumindest einer von der Primärpumpe oder der Konzentratpumpe sind und wobei die Titrationslösung in Schritt f. durch Säure oder Alkali, bereitgestellt von der Primärpumpe und/oder der Konzentratpumpe, eingestellt wird.

10. Verfahren nach Anspruch 7, wobei eine Frequenz einer von der Primärpumpe oder der Konzentratpumpe während der Schritte d. und e. gleichbleibend gehalten wird, wohingegen eine Frequenz der anderen von der Primärpumpe oder der Konzentratpumpe während der Schritte d. und e. variiert wird.

11. System oder Verfahren nach einem der vorstehenden Ansprüche, wobei die Mischvorrichtung eine Mischschleife einschließt.

12. System oder Verfahren nach einem der vorstehenden Ansprüche, wobei die Proteine zumindest zwei Proteine mit einem ersten isoelektrischen Punkt und ein zweites Protein mit einem zweiten isoelektrischen Punkt einschließen.

## Revendications

1. Système de séparation et de purification de protéines comprenant :
a. un dispositif de mélange ;
b. une pompe principale présentant une admission en communication avec une réserve d'acide, d'alcali ou d'eau et une évacuation en communication avec le dispositif de mélange ;
c. une pompe de concentration présentant une admission en communication avec une réserve d'acide ou d'alcali et une évacuation en communication avec le dispositif de mélange ;
d. une colonne de chromatographie liquide en communication avec le dispositif de mélange et garnie d'une résine échangeuse d'ions et chargée de protéines ;
e. une boucle de réinjection de régulation de processus en communication avec le dispositif de mélange, la boucle de réinjection de régulation de processus comportant un régulateur servant de jonction avec une pompe de titrage et un analyseur de pH de processus en ligne, la pompe de titrage présentant une admission en communication avec une réserve d'acide ou d'alcali et une évacuation en communication avec le dispositif de mélange ;
moyennant quoi le régulateur surveille le pH d'une solution dans le dispositif de mélange par le biais de l'analyseur de pH et ajuste l'acidité de la solution en commandant à la pompe de titrage de pomper de l'acide ou de l'alcali de la réserve d'acide ou d'alcali pour l'introduire dans le dispositif de mélange afin de fournir un gradient de pH globalement linéaire à la colonne de chromatographie de manière que les protéines soient séparément éluées de la colonne de chromatographie liquide.

2. Système selon la revendication 1, comprenant en outre une pompe de recirculation dans le dispositif de mélange.

3. Système selon la revendication 1, dans lequel la boucle de réinjection de régulation de processus comporte une pluralité de vannes de titrage automatisées en communication avec l'admission de la pompe de titrage, lesquelles sont commandées par le régulateur.

4. Système selon la revendication 1, dans lequel la pompe principale est une pompe à eau pour injection (WFI, water for injection).

5. Système selon la revendication 3 dans lequel les vannes de titrage sont en communication avec au moins une pompe parmi la pompe principale et la pompe de concentration de manière que le pH de la solution de titrage dans le dispositif de mélange soit ajusté par l'acide ou l'alcali en provenance de la pompe principale et/ou l'acide ou l'alcali en provenance de la pompe de concentration.

6. Système selon la revendication 1, dans lequel le régulateur est un automate programmable ou est configuré sous la forme d'une commande de type PID.

7. Procédé de séparation et de purification de protéines comprenant les étapes consistant à :
a. prévoir un dispositif de mélange, une pompe principale en communication avec le dispositif de mélange, une pompe de concentration en communication avec le dispositif de mélange, une colonne de chromatographie liquide en communication avec le dispositif de mélange et une boucle de réinjection de régulation de processus en communication avec le dispositif de mélange comportant un régulateur servant de jonction avec une pompe de titrage et un analyseur de pH de processus en ligne, la pompe de titrage présentant une admission en communication avec une réserve d'acide ou d'alcali et une évacuation en communication avec le dispositif de mélange ;
b. garnir la colonne de chromatographie liquide d'une résine échangeuse d'ions ;
c. charger la colonne de chromatographie d'une solution protéique contenant des protéines ;
d. fournir un acide ou un alcali au dispositif de mélange au moyen de la pompe principale ;
e. fournir un acide ou un alcali au dispositif de mélange au moyen de la pompe de concentration de manière à créer la solution dans le dispositif de mélange ; et
f. ajuster le pH de la solution dans le dispositif de mélange au moyen du régulateur de la boucle de réinjection, moyennant quoi le régulateur surveille le pH d'une solution présente dans le dispositif de mélange par le biais de l'analyseur de pH et ajuste l'acidité de la solution en commandant à la pompe de titrage de pomper de l'acide ou de l'alcali de la réserve d'acide ou d'alcali pour l'introduire dans le dispositif de mélange afin de fournir un gradient de pH globalement linéaire à la colonne de chromatographie de manière que les protéines soient séparément éluées de la colonne de chromatographie liquide.

8. Procédé selon la revendication 7, dans lequel la boucle de réinjection de régulation de processus comporte une pluralité de vannes de titrage automatisées en communication avec l'admission de la pompe de titrage, lesquelles sont commandées par le régulateur.

9. Procédé selon la revendication 8, dans lequel les vannes de titrage sont en communication avec au moins une pompe parmi la pompe principale et la pompe de concentration et dans lequel la solution de titrage de l'étape f. est ajustée par l'acide ou l'alcali fournis par la pompe principale et/ou la pompe de concentration.

10. Procédé selon la revendication 7, dans lequel la fréquence d'une pompe parmi la pompe principale et la pompe de concentration est maintenue stable au cours des étapes d. et e. tandis que la fréquence de l'autre pompe parmi la pompe principale et la pompe de concentration est variée au cours des étapes d. et e.

11. Système ou procédé selon l'une quelconque des revendications précédentes, dans lequel le dispositif de mélange comporte une boucle de mélange.

12. Système ou procédé selon l'une quelconque des revendications précédentes, dans lequel les protéines comprennent au moins deux protéines présentant un premier point isoélectrique et une deuxième protéine présentant un deuxième point isoélectrique.
